# EUROPEAN PATENT APPLICATION

(11) **EP 4 656 239 A2**
(43) Date of publication of application: **03.12.2025**
(21) Application number: 25209238.2
(22) Date of filing: 14.07.2021
(51) Int. Cl.: A61P 7/02

(54) **AQUEOUS PHARMACEUTICAL COMPOSITION COMPRISING A P2Y12 RECEPTOR ANTAGONIST**

(30) Priority: 15.07.2020 WO PCT/EP2020/069976
(62) Divisional of application: 21739730.6
(71) Applicant: Viatris Asia Pacific Pte. Ltd., Singapore 068864 (SG)
(72) Inventor: BUCHMANN, Stephan, 4056 Basel (CH); FRAICHARD, Amandine, 4123 Allschwil (CH); HERRMANN, Charlyse, 4123 Allschwil (CH); LIENHART, Céline, 4123 Allschwil (CH); VON RAUMER, Markus, 4123 Allschwil (CH); WERK, Tobias, 4125 Riehen (CH)
(74) Representative: Elkington and Fife LLP

(57) **Abstract**

The present invention relates to an aqueous pharmaceutical composition comprising the P2Y12 receptor antagonist 4-((*R*)-2-{[6-((*S*)-3-methoxy-pyrrolidin-1-yl)-2-phenyl-pyrimidine-4-carbonyl]-amino}-3-phosphono-propionyl)-piperazine-1-carboxylic acid butyl ester and a pharmaceutically acceptable buffer and its use as a medicament by parenteral administration.

## Description

The present invention relates to an aqueous pharmaceutical composition comprising the P2Y12 receptor antagonist 4-((*R*)-2-{[6-((*S*)-3-methoxy-pyrrolidin-1-yl)-2-phenyl-pyrimidine-4-carbonyl]-amino}-3-phosphono-propionyl)-piperazine-1-carboxylic acid butyl ester (hereinafter COMPOUND, also known as selatogrel) and a pharmaceutically acceptable buffer; and its use as a medicament by parenteral administration (especially subcutaneous (s.c.) or intradermal administration).

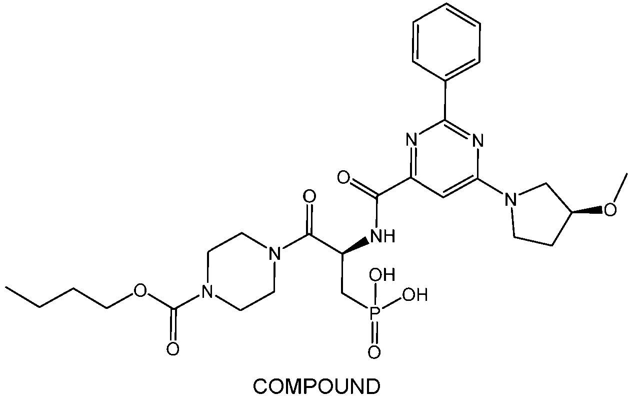

The preparation and the medical use of COMPOUND is described in WO 2009/069100; WO 2018/055016; WO 2018/167139; Baldoni D et al., Clin Drug Investig (2014), 34(11), 807-818; Caroff E et al., J. Med. Chem. (2015), 58, 9133-9153; Storey R F et al., European Heart Journal, ehz807, doi:10.1093/eurheartj/ehz807; and Sinnaeve P R et al, J Am Coll Cardiol (2020), 75 (20), 2588-97 (doi.org/10.1016/j.jacc.2020.03.059).

When vascular integrity is compromised, circulating platelets adhere to the damaged vessel wall and aggregate, forming a plug that seals off the site of injury to prevent blood loss. Autopsy studies demonstrated that rupture of atherosclerotic plaques can lead to uncontrolled platelet thrombus formation and vessel occlusion (Davies MJ et al. (1986) Circulation 73:418-427). Inhibition of platelet aggregation is recognized as an effective strategy for prevention of atherothrombotic events in patients with atherosclerotic disease in the coronary, peripheral, and cerebrovascular circulation (Davi G et al. (2007) N Engl J Med 357:2482-2494).

The ADP receptors P2Y₁ and P2Y₁₂ play a critical role in platelet activation and aggregation (Andre P et al. (2003) J Clin Invest 112:398-406). Inhibition of the P2Y₁₂ receptor is a validated concept for prevention of major adverse cardiovascular events in patients with acute coronary syndromes (ACS) as demonstrated by the thienopyridines ticlopidine, clopidogrel and prasugrel (Franchi F et al. (2015) Nat Rev Cardiol 12:30-47). These drugs following metabolic activation irreversibly block the P2Y₁₂ receptor and platelet function (Antman EM et al. (2004) Circulation 110:588-636; Anderson JL et al. (2011) Circulation 123:e426-579) resulting in increased efficacy and increased bleeding (Wiviott SD et al. (2007) Circulation 116:2923-2932). In studies using P2Y₁₂ knockout mice it was shown that clopidogrel and prasugrel caused more blood loss following tail transection as compared with vehicle indicating that the increased blood loss may be due to off-target effects of the thienopyridines (Andre P et al. (2011) J Pharmacol Exp Ther 338:22-30).

Preclinical studies with the direct-acting and reversibly binding P2Y₁₂ antagonist ticagrelor demonstrated a wider therapeutic window in rat and dog thrombosis models as compared with clopidogrel (van Giezen JJ et al. (2009) Thromb Res 124:565-571; Becker RC et al. (2010) Thromb Haemost 103:535-544). It was argued that the reversibility of the binding of ticagrelor to P2Y₁₂ might account for this difference. In patients, ticagrelor achieved a higher extent of inhibition of ADP-induced platelet aggregation than clopidogrel (Husted S et al. (2006) Eur Heart J 27:1038-1047; Cannon CP et al. (2010) Lancet 375:283-293) and in the pivotal phase III trial (PLATO) in post acute coronary syndrome patients, ticagrelor showed superior efficacy and no significant difference in the risk of major bleeding events to clopidogrel. However, a significant increase in fatal intracranial bleedings and in major or minor bleedings according to the study criteria was reported for ticagrelor (Wallentin L et al. (2009) N Engl J Med 361:1045-1057). COMPOUND was described to cause significantly less blood loss than ticagrelor at equivalent antithrombotic efficacy in rat (Rey M et al. (2017) Pharma Res Per, 5(5), e00338 (doi: 10.1002/prp2.338)).

Several studies confirmed the benefit of earlier administration of inhibitors of platelet function, for example glycoprotein IIb/IIIa inhibitors in patients with ST elevation myocardial infarction (STEMI), especially in those presenting very soon after symptom onset (Van't Hof AW et al. (2008) Lancet 372:537-546; Herrmann HC et al. (2009) JACC Cardiovasc Interv 2:917-924; ten Berg JM et al. (2010) J Am Coll Cardiol 55:2446-2455). Furthermore, various studies and meta-analyses suggested that pretreatment with clopidogrel in patients with STEMI could reduce the rate of ischemic events without excess bleeding (Sabatine MS et al. (2005) JAMA 294:1224-1232; Bellemain-Appaix A et al. (2012) JAMA 308:2507-2516; Zeymer U et al. (2012) Clin Res Cardiol 101:305-312). However, the effectiveness of clopidogrel is limited by its slow onset of action and the variable response (Oliphant CS et al. (2016) J Pharm Pract 29:26-34). The oral P2Y₁₂-receptor antagonists ticagrelor and prasugrel were reported to inhibit platelet function in less than 1 hour (Storey RF et al. (2010) J Am Coll Cardiol 56:1456-1462; Wiviott SD et al. (2007) Circulation 116:2923-2932). However, contradictory studies suggested that the full effect of prasugrel or ticagrelor on platelet function may take several hours in patients with STEMI (Alexopoulos D et al. (2012) Circ Cardiovasc Interv 5:797-804; Valgimigli M et al. (2012) JACC Cardiovasc Interv 5:268-277; Parodi G et al. (2013) J Am Coll Cardiol 61:1601-1606; Montalescot G et al. (2014) N Engl J Med 371:2339). Indeed, in the ATLANTIC clinical trial (Montalescot G et al. (2013) N Engl J Med 369:999-1010) it was found that the two co-primary end points did not differ significantly between a group of patients with ongoing STEMI that has received an early prehospital treatment (in the ambulance) of oral Ticagrelor and a group that has received the treatment only later in the hospital (in the catheterization laboratory). The two co-primary endpoints were (i) the proportion of patients who did not have a 70% or greater resolution of ST-segment elevation before percutaneous coronary intervention (PCI) and (ii) the proportion of patients who did not have Thrombolysis in Myocardial Infarction flow grade 3 in the infarct-related artery at initial angiography. Montalescot et al. stated that besides an extremely short time to PCI in this study "another potential limitation is related to the delayed absorption of orally administered P2Y₁₂ receptor antagonists" and that the "onset of action may have been delayed further by morphine co-administration in half of the study population". There is thus a strong need for a treatment option resulting in a fast and high inhibition of platelet aggregation in patients with, for instance, acute coronary syndromes, wherein the inhibition of platelet aggregation is achieved as early as possible after cardiac symptom onset.

It has been found that this need for fast and high inhibition of platelet aggregation may be fulfilled by subcutaneous administration of COMPOUND to a patient, especially if COMPOUND is administered by the patient or a relative already before the arrival of an ambulance health care provider. In a randomized, double-blind, placebo-controlled clinical study in 48 healthy subjects, it was found that the median time to reach maximum plasma concentration of COMPOUND was only Tₘₐₓ = 0.5 hours after subcutaneous administration at doses between 1 mg and 8 mg, and Tₘₐₓ = 0.75 hours at doses of 16 mg and 32 mg (WO 2018/167139, Table 2, page 33; Juif et al., J. Clin. Pharmacol. (2019) 59(1), 123-130). Even more importantly, a pharmacodynamic effect of more than 85% inhibition of platelet aggregation was observed already 15 minutes after subcutaneous administration of Compound 1 at doses of 8 mg, 16 mg and 32 mg (WO 2018/167139, Table 3, page 34). A fast onset of a pharmacodynamic effect was confirmed in a clinical study in 47 patients with acute myocardial infarction (AMI) (Sinnaeve P R et al, J Am Coll Cardiol (2020), 75 (20), 2588-97 (doi.org/10.1016/j.jacc.2020.03.059)). The authors conclude: "Single-dose subcutaneous administration of selatogrel in patients with AMI was safe and induced a profound, rapid, and dose-related antiplatelet response".

To be suitable for a subcutaneous administration form, the pharmaceutical composition comprising COMPOUND needs to be sufficiently stable for at least 12 months (especially at least 18 months and notably at least 24 months) if stored at about room temperature. Unfortunately, it was found that solutions of COMPOUND in water at lower pH values are not sufficiently stable. For instance, a solution of COMPOUND (32 mg/mL) in water at pH 7 (adjusted with aqueous NaOH solution) contained only 33.5% COMPOUND, but 65.2% (R)-2-(6-((S)-3-methoxypyrrolidin-1-yl)-2-phenylpyrimidine-4-carboxamido)-3-phosphonopropanoic acid (hereinafter DEGRADATION COMPOUND) if stored at 60°C for 1 week (98.6% COMPOUND at t = 0, i.e. immediately after preparation of the solution). The amount of DEGRADATION COMPOUND dropped to 24.4% and 10.0% for corresponding aqueous solutions stored under the same conditions but adjusted to pH 8.0 and 9.0, respectively. In addition, it was found that the pH value of an unbuffered, pH-adjusted, aqueous solution of COMPOUND shifted from pH 9.0 to pH 8.5 after storage of the solution for 2 days at 70°C.

### Description of the figures:

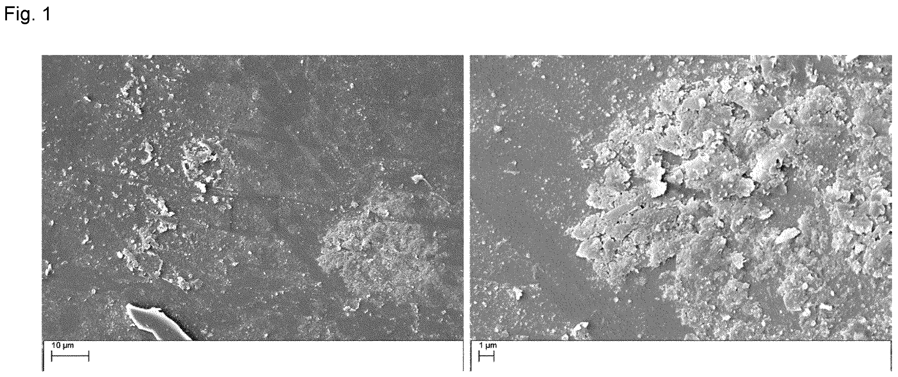

Fig. 1 shows a Scanning Electron Microscopy (SEM) image of the inner part of the barrel of a glass syringe in two different magnifications. The displayed area of the inner part of the barrel was in contact to an aqueous pharmaceutical composition comprising a boric acid buffer for 6 days at 70°C.

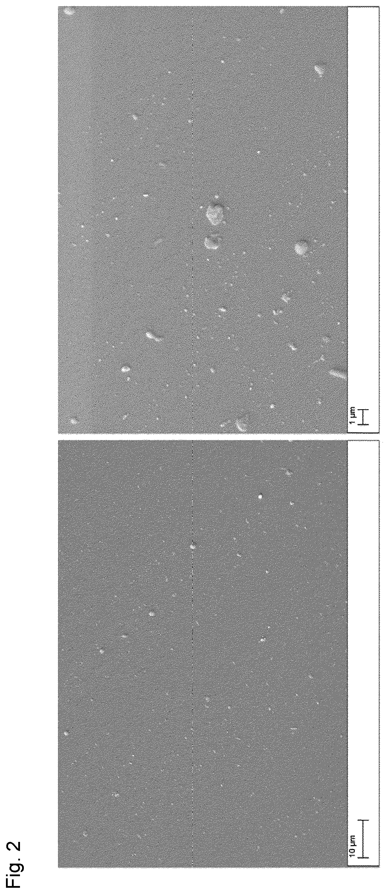

Fig. 2 shows a Scanning Electron Microscopy (SEM) image of the inner part of the barrel of a glass syringe in two different magnifications. The displayed area of the inner part of the barrel was outside of the area that was in contact to an aqueous pharmaceutical composition comprising a boric acid buffer for 6 days at 70°C.

### Description of the invention:

Surprisingly, it has been found that the stability of an aqueous solution of COMPOUND can be significantly improved by buffering the solution at a pH value at or above 8.2.
1) A first embodiment relates to an aqueous pharmaceutical composition comprising:
   - 4-((*R*)-2-{[6-((*S*)-3-methoxy-pyrrolidin-1-yl)-2-phenyl-pyrimidine-4-carbonyl]-amino}-3-phosphono-propionyl)-piperazine-1-carboxylic acid butyl ester (COMPOUND);
   - a pharmaceutically acceptable buffer; and
   - water;
wherein the aqueous pharmaceutical composition has a pH value between 8.2 and 12.0.

It is to be understood that COMPOUND may be used in the preparation of the aqueous pharmaceutical composition in any form. For instance, COMPOUND may be used in any amorphous or crystalline form, in anhydrous form or in form of a hydrate or a pharmaceutically acceptable solvate, in form of the free compound (i.e. non-salt form), in form of a pharmaceutically acceptable salt or in any mixture of such forms. In a preferred embodiment, COMPOUND is used in the preparation of the aqueous pharmaceutical composition in form of the hydrochloride salt (especially in a polymorphic form disclosed in WO 2018/055016). In another preferred embodiment, COMPOUND is used in the preparation of the aqueous pharmaceutical composition in form of the di-sodium salt. In still another preferred embodiment, COMPOUND is used in the preparation of the aqueous pharmaceutical composition in form of the free compound (non-salt form). It is preferred that COMPOUND, or a pharmaceutically acceptable salt thereof, is used in the preparation of the present pharmaceutical compositions in essentially pure form. The amount of COMPOUND may be adjusted taking into account the actual chemical purity, or the presence of a salt form and/or of a solvate or hydrate, of COMPOUND.

It is further to be understood that the pH value of the aqueous pharmaceutical composition may be adjusted to the target value with a pharmaceutically acceptable base. The pharmaceutically acceptable base used in the preparation of the aqueous pharmaceutical composition may be a pharmaceutically acceptable strong base, a pharmaceutically acceptable weak base different from the basic component of the pharmaceutically acceptable buffer, or the basic component of the pharmaceutically acceptable buffer that is used in the aqueous pharmaceutical composition. It is understood that the basic component of the pharmaceutically acceptable buffer may be in essentially pure form or in a mixture with the acidic component of the pharmaceutically acceptable buffer, wherein the ratio between the basic and the acidic component is sufficiently high to adjust the pH value of the aqueous pharmaceutical composition to the target value. In a preferred embodiment, the pharmaceutically acceptable base is a pharmaceutically acceptable strong base, or the basic component of the pharmaceutically acceptable buffer that is used in the aqueous pharmaceutical composition. In a most preferred embodiment, the pharmaceutically acceptable base is a pharmaceutically acceptable strong base (especially sodium hydroxide). To adjust the pH value of the aqueous pharmaceutical composition, the pharmaceutically acceptable base may be used in essentially pure form without a solvent, or as a solution in a pharmaceutically acceptable solvent or solvent mixture (especially a solution in water). In a most preferred embodiment, a solution of sodium hydroxide in water is used as the pharmaceutically acceptable base.

Only in the case that the pH value of the aqueous pharmaceutical composition before pH adjustment is higher than the targeted pH value, a pharmaceutically acceptable acid, especially a pharmaceutically acceptable strong acid, such as aqueous hydrochloride acid, may be used for pH adjustment.

It is known in the art that the pH value of an aqueous pharmaceutical composition may change over time, especially if stored for one or several years. Thus, if an aqueous pharmaceutical composition is described and/or claimed to have a pH value between a specific lower limit and a specific upper limit, this means that the pH value of the aqueous pharmaceutical composition is in the given range defined by the specific lower limit and the specific upper limit at least immediately (for instance 1 hour or 2 hours) after final preparation of the aqueous pharmaceutical composition (i.e. after all ingredients are added to the aqueous pharmaceutical composition). Preferably the pH value remains in the given range defined by the specific lower limit and the specific upper limit for at least 6 months (preferably at least 12 months and most preferably at least 18 months) after final preparation of the aqueous pharmaceutical composition.

The term "pharmaceutically acceptable buffer" refers to a mixture of a weak Brønsted base (the basic component of the pharmaceutically acceptable buffer) and its conjugated acid (the acidic component of the pharmaceutically acceptable buffer), wherein both, the weak base and the conjugated acid exhibit minimal undesired toxicological effects. The weak base may be an inorganic or organic base and may be in the form of a neutral molecule or in form of a salt comprising a protonatable anion. For avoidance of doubt, in the present application boric acid is understood to be a weak Brønsted acid and a boric acid buffer is thus comprised in the definition of a pharmaceutically acceptable buffer. The pharmaceutically acceptable buffer is selected from the group of pharmaceutically acceptable buffer with a pKa value in water at 25°C in the range of the targeted pH value ±1.5 (pH-1.5 ≤ pKa ≤ pH+1.5); preferred are pharmaceutically acceptable buffer with a pKa value in water at 25°C in the range of the targeted pH value ±1 (pH-1 ≤ pKa ≤ pH+1). For instance, if the target value for the aqueous pharmaceutical composition is pH = 9.0, the pharmaceutically acceptable buffer is selected from pharmaceutically acceptable buffer with a pKa value in water at 25°C in the range between 7.5 and 10.5 (and preferably between 8.0 and 10.0). Examples of pharmaceutically acceptable buffer are ammonium buffer, boric acid buffer, carbonate buffer, phosphate buffer, arginine buffer, glycine buffer, histidine buffer, meglumine buffer (also known as N-methyl-D-glucamine buffer), 2-aminoethanol buffer (also known as monoethanolamine buffer), bis(2-hydroxyethyl)amine buffer (also known as diethanolamine buffer), and tris(hydroxymethyl)aminomethane buffer (also known as tromethamine or Tris buffer). It is preferred that the pharmaceutically acceptable buffer is different from phosphate buffer. Most preferred are boric acid buffer and arginine buffer.

The pharmaceutically acceptable buffer may be obtained from: ammonium chloride, ammonium bromide or ammonia (e.g. as solution in water) for ammonium buffer; boric acid or sodium tetraborate for boric acid buffer; sodium hydrogencarbonate or sodium carbonate for carbonate buffer; sodium dihydrogenphosphate or disodium hydrogenphosphate for phosphate buffer; arginine or arginine hydrochloride for arginine buffer; glycine or glycine hydrochloride for glycine buffer; histidine or histidine hydrochloride for histidine buffer; N-methyl-D-glucamine or N-methyl-D-glucamine hydrochloride for meglumine buffer; 2-aminoethanol or 2-aminoethanol hydrochloride for 2-aminoethanol buffer; bis(2-hydroxyethyl)amine or bis(2-hydroxyethyl)amine hydrochloride for bis(2-hydroxyethyl)amine buffer; tris(hydroxymethyl)aminomethane or tris(hydroxymethyl) aminomethane hydrochloride for tris(hydroxymethyl)aminomethane buffer; or from any other suitable starting material.

It is understood that any reference to one of the amino acids arginine or histidine refers to the respective amino acid in its D-form, its L-form or any mixture (including a racemic mixture) thereof; and especially to the respective amino acid in its enantiomerically essentially pure L-form.

The term "pharmaceutically acceptable salt" refers to salts that retain the desired biological activity of the subject compound and exhibit minimal undesired toxicological effects. Such salts include inorganic or organic acid and/or base addition salts depending on the presence of basic and/or acidic groups in the subject compound. For reference see for example 'Handbook of Pharmaceutical Salts. Properties, Selection and Use.', P. Heinrich Stahl, Camille G. Wermuth (Eds.), Wiley-VCH, 2008 and 'Pharmaceutical Salts and Co-crystals', Johan Wouters and Luc Quéré (Eds.), RSC Publishing, 2012.

A preferred pharmaceutically acceptable salt of COMPOUND is the hydrochloride salt of COMPOUND (acid addition salt). Other preferred pharmaceutically acceptable salts of COMPOUND are the alkali metal and alkaline earth metal salts of COMPOUND (base addition salts); especially the sodium and potassium salts and notably the sodium salts of COMPOUND. It is understood that in base addition salts one or both acidic protons of COMPOUND may be replaced by cations of the base. The most preferred base addition salt of COMPOUND is the disodium salt of COMPOUND.

In the context of adjusting the pH value of the aqueous pharmaceutical composition, the term "pharmaceutically acceptable base" refers to a Brønsted base whose reaction products from the adjustment of the pH value (e.g. a sodium salt and water if sodium hydroxide is used as base) exhibit minimal undesired toxicological effects. The term "pharmaceutically acceptable strong base" refers to a "pharmaceutically acceptable base" that is completely or almost completely ionized or dissociated if dissolved in water. Examples of pharmaceutically acceptable strong bases are hydroxide salts of alkali or alkaline earth metals; especially sodium hydroxide or potassium hydroxide; and notably sodium hydroxide.

A solvate of COMPOUND is a "pharmaceutically acceptable solvate" if the solvent forming the solvate exhibits minimal undesired toxicological effects.

The term "target value" in relation to the pH value of the aqueous pharmaceutical composition (or alternatively "targeted pH value") means the pH value to which the aqueous pharmaceutical composition should be adjusted. Preferably, the pH value of the aqueous pharmaceutical composition immediately after its final preparation is equal to the target value.

The term "essentially", for example when used in a term such as "essentially pure" is understood in the context of the present invention to mean especially that the respective composition / component / compound / stereoisomer etc. consists in an amount of at least 90, especially of at least 95, and notably of at least 99 per cent by weight of the respective pure composition / component / compound / stereoisomer etc.. Especially, the term "consisting essentially of" is understood in the context of the present invention to mean that the respective composition consists in an amount of at least 90, especially of at least 95, notably of at least 99, and in particular in an amount of 100 per cent by weight (i.e. in the meaning of "consisting of") of the respective composition in the amounts as explicitly stated in the respective embodiment.

The total weight percent of the aqueous pharmaceutical composition as defined in embodiment 1), and embodiments 2) to 60) below is 100.

For avoidance of any doubt, it is well understood that pharmaceutical compositions as defined in embodiment 1) and 2) to 6) and 10) to 60) may additionally comprise further conventional ingredients and/ or additives, which may be used alone or in combination.

Reference is made to the extensive literature on the subject for these and other pharmaceutically acceptable excipients and procedures mentioned herein, see for example R.C. Rowe, P.J. Seskey, S.C. Owen, Handbook of Pharmaceutical Excipients, 5th edition, Pharmaceutical Press 2006; Remington, The Science and Practice of Pharmacy, 21st Edition (2005), Part 5, "Pharmaceutical Manufacturing" [published by Lippincott Williams & Wilkins]; and Nema, Brendel, Excipients and Their Role in Approved Injectable Products: Current Usage and Future Directions, PDA J Pharm Sci and Tech (2011), 65, 287-332.

Examples of such conventional ingredients or additives are tonicity modifiers such as salts (e.g. NaCl, KCl, MgCl₂, CaCl₂). Further conventional ingredients or additives are for example antimicrobial preservatives such as used e.g. in bacteriostatic water for injection. An example is benzyl alcohol.

It is understood that the aqueous pharmaceutical composition may in addition to water further comprise up to 20 ww% (especially up to 10 ww% and notably up to 5 ww%) of a pharmaceutically acceptable solvent or a mixture of pharmaceutically acceptable solvents. The term "pharmaceutically acceptable solvent" refers to a solvent that exhibits minimal undesired toxicological effects. Representative examples of pharmaceutically acceptable solvents are polyethylene glycol, polysorbate, DMSO and NMP. In a preferred embodiment, the aqueous pharmaceutical composition does not contain a pharmaceutically acceptable solvent in addition to water (other than trace amounts of residual solvent present in an ingredient used in the preparation of the aqueous pharmaceutical composition).

The term "pharmaceutical composition" is interchangeable with the term "formulation".

Unless used regarding temperatures, the term "about" placed before a numerical value "X" refers in the current application to an interval extending from X minus 10% of X to X plus 10% of X, and preferably to an interval extending from X minus 5% of X to X plus 5% of X (wherein it is well understood that the lower limit is equal or above 0% and the higher limit is equal or below 100%). In the particular case of temperatures, the term "about" placed before a temperature "Y" refers in the current application to an interval extending from the temperature Y minus 10 °C to Y plus 10 °C; and preferably to an interval extending from Y minus 5 °C to Y plus 5 °C.

Whenever the word "between" or "to" is used to describe a numerical range, it is to be understood that the end points of the indicated range are explicitly disclosed and included in the range. For example: if a pH value is described to be between 8.2 and 12.0 (or 8.2 to 12.0), this means that the end points 8.2 and 12.0 are included in the range; or if a variable is defined as being an integer between 1 and 4 (or 1 to 4), this means that the variable is the integer 1, 2, 3, or 4.

The expression "ww%" (or % (w/w)) refers to a percentage by weight compared to the total weight of the composition considered. If not explicitly stated otherwise, the considered total weight is the total weight of the aqueous pharmaceutical composition. In case a certain value is given as % value, in absence of further specification such value refers to ww%. The expression (wt/wt) relating to a ratio refers to a ratio by weight of the respective components.

Further embodiments of the invention are presented hereinafter:
2) In another embodiment, the invention relates to an aqueous pharmaceutical composition comprising:
   - COMPOUND in an amount between 0.1 to 20 ww%;
   - a pharmaceutically acceptable buffer; and
   - water;
   wherein the aqueous pharmaceutical composition has a pH value between 8.5 and 11.0.
3) In another embodiment, the invention relates to an aqueous pharmaceutical composition according to any one of embodiments 1) or 2), wherein the sum of the amount of COMPOUND, of the amount of the pharmaceutically acceptable buffer and of the amount of water is at least 80 ww% of the aqueous pharmaceutical composition.
4) In another embodiment, the invention relates to an aqueous pharmaceutical composition according to any one of embodiments 1) or 2), wherein the sum of the amount of COMPOUND, of the amount of the pharmaceutically acceptable buffer and of the amount of water is at least 90 ww% of the aqueous pharmaceutical composition.
5) In another embodiment, the invention relates to an aqueous pharmaceutical composition according to any one of embodiments 1) or 2), wherein the sum of the amount of COMPOUND, of the amount of the pharmaceutically acceptable buffer and of the amount of water is at least 95 ww% of the aqueous pharmaceutical composition.
6) In another embodiment, the invention relates to an aqueous pharmaceutical composition according to any one of embodiments 1) or 2), wherein the sum of the amount of COMPOUND, of the amount of the pharmaceutically acceptable buffer and of the amount of water is at least 98 ww% of the aqueous pharmaceutical composition.
7) In another embodiment, the invention relates to an aqueous pharmaceutical composition according to embodiment 1), consisting essentially of:
   - COMPOUND in an amount between 0.1 to 20 ww%;
   - a pharmaceutically acceptable buffer;
   - water; and
   - optionally an inorganic salt of an alkali metal or an alkaline earth metal (preferably of lithium, sodium, potassium, magnesium or calcium and most preferably of sodium or potassium);
   wherein the aqueous pharmaceutical composition has a pH value between 8.5 and 11.0.

The term "optionally", as used in in the context of an optionally present excipient, such as an inorganic salt, means that the excipient may be absent or present in the aqueous pharmaceutical composition.
8) In another embodiment, the invention relates to an aqueous pharmaceutical composition according to embodiment 1), consisting essentially of:
   - COMPOUND in an amount between 0.7 to 7.0 ww%;
   - a pharmaceutically acceptable buffer selected from a boric acid buffer or an arginine buffer;
   - water; and
   - optionally an inorganic salt of an alkali metal or an alkaline earth metal (preferably of lithium, sodium, potassium, magnesium or calcium and most preferably of sodium or potassium);
   wherein the aqueous pharmaceutical composition has a pH value between 8.7 and 9.5.
9) In another embodiment, the invention relates to an aqueous pharmaceutical composition according to any one of embodiments 1) to 6), wherein the pharmaceutical composition is obtainable by:
   - addition of 4-((R)-2-{[6-((S)-3-methoxy-pyrrolidin-1-yl)-2-phenyl-pyrimidine-4-carbonyl]-amino}-3-phosphono-propionyl)-piperazine-1-carboxylic acid butyl ester hydrochloride to a solution of arginine or boric acid, and optionally sodium chloride, in water; and
   - addition of an aqueous solution of a pharmaceutically acceptable strong base (especially sodium hydroxide) until the targeted pH value between 8.2 and 12.0 is reached;
   wherein the amount of COMPOUND in the final pharmaceutical composition is between 0.1 ww% and 20 ww%.
10) In another embodiment, the invention relates to an aqueous pharmaceutical composition according to any one of embodiments 1) or 3) to 6), wherein the amount of COMPOUND is between 0.1 ww% and 20 ww%.
11) In another embodiment, the invention relates to an aqueous pharmaceutical composition according to any one of embodiments 1) to 9), wherein the amount of COMPOUND is between 0.7 ww% and 7.0 ww%. Lower limits of the amount of COMPOUND are 0.7 ww%, 1.0 ww% and 2.0 ww%, upper limits are 7.0 ww%, 5.0 ww% and 4.0 ww%. It is to be understood that each lower limit can be combined with each upper limit. Hence all combinations shall herewith be disclosed. A preferred amount of COMPOUND is from 1.0 ww% to 5.0 ww%.
12) In another embodiment, the invention relates to an aqueous pharmaceutical composition according to any one of embodiments 1) to 9), wherein the amount of COMPOUND is between 2.4 ww% and 3.8 ww%.
13) In another embodiment, the invention relates to an aqueous pharmaceutical composition according to any one of embodiments 1) or 3) to 6), wherein the mass concentration of COMPOUND in the aqueous pharmaceutical composition is between 6 mg/mL and 60 mg/mL, preferably between 15 mg/mL and 45 mg/mL and most preferably between 25 mg/mL and 40 mg/mL.
14) In another embodiment, the invention relates to an aqueous pharmaceutical composition according to any one of embodiments 1) to 9), wherein the mass concentration of COMPOUND in the aqueous pharmaceutical composition is about 32 mg/mL.
15) In another embodiment, the invention relates to an aqueous pharmaceutical composition according to any one of embodiments 1) to 7) or 10) to 14), wherein the pharmaceutically acceptable buffer has a pKa value in water at 25°C between 8.0 and 11.0. Lower limits of the pKa value are 8.0, 8.5 and 8.8, upper limits are 11.0, 10.0 and 9.5. It is to be understood that each lower limit can be combined with each upper limit. Hence all combinations shall herewith be disclosed. It is further to be understood that in the case of a di- or triprotic acid only one of the two or three pKa values has to be in the above-mentioned range.
16) In another embodiment, the invention relates to an aqueous pharmaceutical composition according to any one of embodiments 1) to 7) or 10) to 14), wherein the pharmaceutically acceptable buffer has a pKa value in water at 25°C between 8.8 and 9.5.
17) In another embodiment, the invention relates to an aqueous pharmaceutical composition according to any one of embodiments 1) to 7) or 10) to 14), wherein the pharmaceutically acceptable buffer is selected from ammonium buffer, boric acid buffer, carbonate buffer, phosphate buffer, arginine buffer, glycine buffer, histidine buffer, meglumine buffer, or tris(hydroxymethyl)aminomethane buffer (preferably from ammonium buffer, boric acid buffer, carbonate buffer, arginine buffer, glycine buffer, histidine buffer, meglumine buffer, or tris(hydroxymethyl)aminomethane buffer).
18) In another embodiment, the invention relates to an aqueous pharmaceutical composition according to any one of embodiments 1) to 14), wherein the pharmaceutically acceptable buffer is selected from boric acid buffer, or arginine buffer (preferably boric acid buffer, or L-arginine buffer).
19) In another embodiment, the invention relates to an aqueous pharmaceutical composition according to any one of embodiments 1) to 14), wherein the pharmaceutically acceptable buffer is boric acid buffer.
20) In another embodiment, the invention relates to an aqueous pharmaceutical composition according to any one of embodiments 1) to 14), wherein the pharmaceutically acceptable buffer is arginine buffer.
21) In another embodiment, the invention relates to an aqueous pharmaceutical composition according to any one of embodiments 1) to 14), wherein the pharmaceutically acceptable buffer is L-arginine buffer.

The term "L-arginine buffer" refers to an arginine buffer wherein the enantiomeric ratio between the L-form and the D-form is at least 90/10, preferably at least 95/5, more preferably at least 99/1, and most preferably at least 99.9/0.1.

22) In another embodiment, the invention relates to an aqueous pharmaceutical composition according to any one of embodiments 1) to 21), wherein the molar concentration of the pharmaceutically acceptable buffer in the aqueous pharmaceutical composition is between 10 mmol/L and 300 mmol/L. Lower limits of the molar concentration of the pharmaceutically acceptable buffer in the aqueous pharmaceutical composition are 10 mmol/L, 25 mmol/L, and **40** mmol/L, upper limits are 300 mmol/L, 200 mmol/L, and 100 mmol/L. It is to be understood that each lower limit can be combined with each upper limit. Hence all combinations shall herewith be disclosed.

The term "molar concentration of the pharmaceutically acceptable buffer" refers to the sum of the molar concentrations of all different protonated and deprotonated components of the buffer system in the respective aqueous pharmaceutical composition. The "molar concentration of the pharmaceutically acceptable buffer" may be calculated by dividing the amount of the added buffer component during preparation of the aqueous pharmaceutical composition (in moles) by the total volume of the finally prepared aqueous pharmaceutical composition (in litres). In the specific case of a monoprotic buffer, the term "molar concentration of the pharmaceutically acceptable buffer" refers to the sum of the molar concentration of the basic component of the pharmaceutically acceptable buffer and of the molar concentration of the acidic component of the pharmaceutically acceptable buffer in the respective aqueous pharmaceutical composition.

It is preferred that the buffer capacity of the pharmaceutically acceptable buffer in the aqueous pharmaceutical composition is between 10 mM/pH and 170 mM/pH. Lower limits of the buffer capacity are 10 mM/pH, 15 mM/pH, and 20 mM/pH; upper limits are 170 mM/pH, 100 mM/pH, and 60 mM/pH. It is to be understood that each lower limit can be combined with each upper limit. Hence all combinations shall herewith be disclosed. Preferably the buffer capacity is between 20 mM/pH and 60 mM/pH.
23) In another embodiment, the invention relates to an aqueous pharmaceutical composition according to any one of embodiments 1) to 21), wherein the molar concentration of the pharmaceutically acceptable buffer in the aqueous pharmaceutical composition is between 25 mmol/L and 200 mmol/L.
24) In another embodiment, the invention relates to an aqueous pharmaceutical composition according to any one of embodiments 1) to 21), wherein the molar concentration of the pharmaceutically acceptable buffer in the aqueous pharmaceutical composition is between 40 mmol/L and 100 mmol/L.
25) In another embodiment, the invention relates to an aqueous pharmaceutical composition according to any one of embodiments 1) to 21), wherein the molar concentration of the pharmaceutically acceptable buffer in the aqueous pharmaceutical composition is about 60 mmol/L.
26) In another embodiment, the invention relates to an aqueous pharmaceutical composition according to any one of embodiments 1) to 25), wherein the water is water for injection (WFI), sterile water for injection (SWFI), or bacteriostatic water for injection (BWFI).
27) In another embodiment, the invention relates to an aqueous pharmaceutical composition according to any one of embodiments 1) to 25), wherein the water is water for injection (WFI).
28) In another embodiment, the invention relates to an aqueous pharmaceutical composition according to any one of embodiments 1) to 7) or 9) to 27), wherein the aqueous pharmaceutical composition has a pH value between 8.5 and 11.0. Lower limits of the pH value of the aqueous pharmaceutical composition are 8.5, 8.7, and 8.9; upper limits are 11.0, 9.5, and 9.3. It is to be understood that each lower limit can be combined with each upper limit. Hence all combinations shall herewith be disclosed.
29) In another embodiment, the invention relates to an aqueous pharmaceutical composition according to any one of embodiments 1) to 27), wherein the aqueous pharmaceutical composition has a pH value between 8.7 and 9.5.
30) In another embodiment, the invention relates to an aqueous pharmaceutical composition according to any one of embodiments 1) to 27), wherein the aqueous pharmaceutical composition has a pH value between 8.9 and 9.3.
31) In another embodiment, the invention relates to an aqueous pharmaceutical composition according to any one of embodiments 1) to 30), wherein the inorganic salt, if present, is selected from salts of formula MX, wherein M represents lithium, sodium, or potassium and X represents chlorine or bromine; or MX₂, wherein M represents magnesium or calcium and X represents chlorine or bromine.
32) In another embodiment, the invention relates to an aqueous pharmaceutical composition according to any one of embodiments 1) to 30), wherein the inorganic salt, if present, is selected from sodium chloride, potassium chloride, magnesium chloride, calcium chloride, or any mixture thereof (and preferably sodium chloride, potassium chloride, or any mixture thereof; and most preferably sodium chloride).
33) In another embodiment, the invention relates to an aqueous pharmaceutical composition according to any one of embodiments 1) to 30), wherein the aqueous pharmaceutical composition comprises sodium chloride.

The inorganic salt (especially sodium chloride) is used to adjust the osmolality of the aqueous pharmaceutical composition to a value ≥ 230 mOsm/kg and notably to a value between 230 mOsm/kg and 1000 mOsm/kg. Lower limits of the osmolality of the aqueous pharmaceutical composition are 230 mOsm/kg, 250 mOsm/kg, and 270 mOsm/kg; upper limits are 1000 mOsm/kg, 500 mOsm/kg, and 330 mOsm/kg. It is to be understood that each lower limit can be combined with each upper limit. Hence all combinations shall herewith be disclosed. Preferably the osmolality is between 270 mOsm/kg and 330 mOsm/kg.
34) In another embodiment, the invention relates to an aqueous pharmaceutical composition according to any one of embodiments 1) to 33), wherein the aqueous pharmaceutical composition is in a container.
35) In another embodiment, the invention relates to an aqueous pharmaceutical composition according to embodiment 34), wherein the container is selected from a vial, an ampoule, a cartridge, or a syringe.
36) In another embodiment, the invention relates to an aqueous pharmaceutical composition according to embodiment 34), wherein the container is a syringe.
37) In another embodiment, the invention relates to an aqueous pharmaceutical composition according to any one of embodiments 34) to 36), wherein the container is a glass container (preferably a glass syringe).
38) In another embodiment, the invention relates to an aqueous pharmaceutical composition according to any one of embodiments 34) to 37), wherein the container has an inner volume between 0.1 mL and 5.0 mL. Lower limits of the inner volume are 0.1 mL, 0.3 mL, 0.5 mL and 0.8 mL, upper limits are 5.0 mL, 3.0 mL, 2.3 mL and 1.0 mL. It is to be understood that each lower limit can be combined with each upper limit. Hence all combinations shall herewith be disclosed.
39) In another embodiment, the invention relates to an aqueous pharmaceutical composition according to any one of embodiments 34) to 37), wherein the container has an inner volume between 0.5 mL and 2.3 mL.
40) In another embodiment, the invention relates to an aqueous pharmaceutical composition according to any one of embodiments 34) to 39), wherein the amount of the aqueous pharmaceutical composition in the container corresponds to the amount of the unit dose.

The term "unit dose" means the amount of the aqueous pharmaceutical composition that is administered and/or is to be administered to a patient in a single dose.

The term "corresponds", when used in the context of "corresponds to the amount of the unit dose", means that the amount of the aqueous pharmaceutical composition equals the amount of the unit dose plus a small addition to compensate for a loss of aqueous pharmaceutical composition during administration, such loss being caused for instance by the dead volume of the respective injection device (e.g. a syringe, an autoinjector, a medical pump or the like).
41) In another embodiment, the invention relates to an aqueous pharmaceutical composition according to embodiment 40), wherein the volume of the aqueous pharmaceutical composition in the container is between 0.1 mL and 3.0 mL. Lower limits of the volume of the aqueous pharmaceutical composition in the container are 0.1 mL, 0.2 mL, 0.3 mL and 0.4 mL, upper limits are 3.0 mL, 1.5 mL, 1.0 mL and 0.7 mL. It is to be understood that each lower limit can be combined with each upper limit. Hence all combinations shall herewith be disclosed.
42) In another embodiment, the invention relates to an aqueous pharmaceutical composition according to embodiment 40), wherein the volume of the aqueous pharmaceutical composition in the container is between 0.3 mL and 1.0 mL (preferably between 0.4 mL and 0.7 mL).
43) In another embodiment, the invention relates to an aqueous pharmaceutical composition according to any one of embodiments 40) to 42), wherein the amount of COMPOUND in the aqueous pharmaceutical composition in the container is between 4.0 mg and 32.0 mg. Lower limits of the amount of COMPOUND in the aqueous pharmaceutical composition in the container are 4.0 mg, 7.0 mg, 10.0 mg and 14.0 mg, upper limits are 32.0 mg, 28.0 mg, 24.0 mg and 20.0 mg. It is to be understood that each lower limit can be combined with each upper limit. Hence all combinations shall herewith be disclosed.
44) In another embodiment, the invention relates to an aqueous pharmaceutical composition according to any one of embodiments 40) to 42), wherein the amount of COMPOUND in the aqueous pharmaceutical composition in the container is between 14.0 mg and 20.0 mg.
45) In another embodiment, the invention relates to an aqueous pharmaceutical composition according to any one of embodiment 34) to 39), wherein the amount of the aqueous pharmaceutical composition in the container corresponds to two or more (up to a maximum of ten) unit doses (preferably two to five and most preferably two or three unit doses).
46) In another embodiment, the invention relates to an aqueous pharmaceutical composition according to any one of embodiments 34) to 45), wherein the container is inserted in an autoinjector device (preferably a pen-injector device).
47) In another embodiment, the invention relates to an aqueous pharmaceutical composition according to any one of embodiments 1) to 46), wherein the aqueous pharmaceutical composition is an emulsion, a suspension or a solution.
48) In another embodiment, the invention relates to an aqueous pharmaceutical composition according to any one of embodiments 1) to 46), wherein the aqueous pharmaceutical composition is a solution.
49) In another embodiment, the invention relates to an aqueous pharmaceutical composition according to any one of embodiments 1) to 48), wherein the aqueous pharmaceutical composition is sterile.
50) In another embodiment, the invention relates to an aqueous pharmaceutical composition according to any one of embodiments 1) to 49), wherein the aqueous pharmaceutical composition is pyrogen-free.
51) In another embodiment, the invention relates to an aqueous pharmaceutical composition according to any one of embodiments 1) to 50), wherein the amount of COMPOUND in the aqueous pharmaceutical composition after storage for 1 year at 25°C is at least 80% (preferably at least 85% and most preferably at least 90%) of the amount of COMPOUND in the aqueous pharmaceutical composition immediately (for instance 1 hour or 2 hours) after final preparation of the aqueous pharmaceutical composition (i.e. after all ingredients are added to the aqueous pharmaceutical composition).
52) In another embodiment, the invention relates to an aqueous pharmaceutical composition according to any one of embodiments 1) to 50), wherein the amount of COMPOUND in the aqueous pharmaceutical composition after storage for 2 years at 25°C is at least 80% (preferably at least 85% and most preferably at least 90%) of the amount of COMPOUND in the aqueous pharmaceutical composition immediately (for instance 1 hour or 2 hours) after final preparation of the aqueous pharmaceutical composition (i.e. after all ingredients are added to the aqueous pharmaceutical composition).
53) In another embodiment, the invention relates to an aqueous pharmaceutical composition according to any one of embodiments 1) to 50), wherein the amount of COMPOUND in the aqueous pharmaceutical composition after storage for 3 years at 25°C is at least 80% (preferably at least 85% and most preferably at least 90%) of the amount of COMPOUND in the aqueous pharmaceutical composition immediately (for instance 1 hour or 2 hours) after final preparation of the aqueous pharmaceutical composition (i.e. after all ingredients are added to the aqueous pharmaceutical composition).
54) In another embodiment, the invention relates to an aqueous pharmaceutical composition according to any one of embodiments 1) to 50), wherein the amount of COMPOUND in the aqueous pharmaceutical composition after storage for 18 months at 30°C is at least 80% (preferably at least 85% and most preferably at least 90%) of the amount of COMPOUND in the aqueous pharmaceutical composition immediately (for instance 1 hour or 2 hours) after final preparation of the aqueous pharmaceutical composition (i.e. after all ingredients are added to the aqueous pharmaceutical composition).
55) In another embodiment, the invention relates to an aqueous pharmaceutical composition according to any one of embodiments 1) to 50), wherein the amount of COMPOUND in the aqueous pharmaceutical composition after storage for 2 years at 30°C is at least 80% (preferably at least 85% and most preferably at least 90%) of the amount of COMPOUND in the aqueous pharmaceutical composition immediately (for instance 1 hour or 2 hours) after final preparation of the aqueous pharmaceutical composition (i.e. after all ingredients are added to the aqueous pharmaceutical composition).
56) In another embodiment, the invention relates to an aqueous pharmaceutical composition according to any one of embodiments 1) to 50), wherein the amount of COMPOUND in the aqueous pharmaceutical composition after storage for 6 month at 2°C to 8°C (especially 5°C) and subsequently for 2 years at 25°C is at least 80% (preferably at least 85% and most preferably at least 90%) of the amount of COMPOUND in the aqueous pharmaceutical composition immediately (for instance 1 hour or 2 hours) after final preparation of the aqueous pharmaceutical composition (i.e. after all ingredients are added to the aqueous pharmaceutical composition).

It has been found that COMPOUND and/or the main degradation product of COMPOUND ((R)-2-(6-((S)-3-methoxypyrrolidin-1-yl)-2-phenylpyrimidine-4-carboxamido)-3-phosphonopropanoic acid) can form covalently bound adducts to the pharmaceutically acceptable buffer used in the aqueous pharmaceutical composition. Such adducts are disadvantageous, inter alia as their toxicological profiles are unknown. Thus, additional toxicological studies may be required, or toxicologically relevant findings may be identified. For instance, the U.S. Food and Drug Administration (FDA) requires a toxicological profiling for all impurities (such as adducts) that are present in the pharmaceutical composition in an amount above 0.5%. The lowest amounts of adducts have been found with arginine buffer, boric acid buffer and carbonate buffer.
57) In another embodiment, the invention relates to an aqueous pharmaceutical composition according to any one of embodiments 1) to 56), wherein the ratio between (i) the amount of a covalently bound adduct of the pharmaceutically acceptable buffer to COMPOUND and (ii) the amount of COMPOUND in the aqueous pharmaceutical composition is less than 1 to 250 (preferably less than 1 to 500) after storage of the aqueous pharmaceutical composition for 2 years at 25°C.
58) In another embodiment, the invention relates to an aqueous pharmaceutical composition according to any one of embodiments 1) to 56), wherein the ratio between (i) the amount of a covalently bound adduct of the pharmaceutically acceptable buffer to ((R)-2-{[6-((S)-3-methoxy-pyrrolidin-1-yl)-2-phenyl-pyrimidine-4-carbonyl]-amino}-3-phosphono-propanoic acid and (ii) the amount of COMPOUND in the aqueous pharmaceutical composition is less than 1 to 250 (preferably less than 1 to 500) after storage of the aqueous pharmaceutical composition for 2 years at 25°C.

In the context of covalently bound adducts, the term "amount" refers to the number of molecules, wherein the number of molecules may be given in any unit directly related to the number of molecules (such as "mol").

In addition, it has been found that, depending on the used pharmaceutically acceptable buffer, the aqueous pharmaceutical composition may modify the surface of glass. This modification may be caused by glass corrosion and/or delamination and results in an increased roughness of the glass surface and/or the occurrence of particles in the aqueous pharmaceutical composition, especially after storage of the aqueous pharmaceutical composition in a glass container such as a glass syringe. The increased roughness and/or the occurrence of particles has several disadvantages. For instance, in case a glass syringe is used for storage of the aqueous pharmaceutical composition and (after storage) for administration to a patient, the occurrence of particles may result in a risk for the patient if such glass particles are injected to the patient (especially if accidentally injected into a vein). An increased roughness of the glass surface of the inner wall of the syringe may result in an increase of the force that is needed to move the plunger/piston within the barrel of the syringe. Especially if the glass syringe is used in an autoinjector device (such as a pen-injector device) this may result in a malfunction of the autoinjector device. Compared to aqueous pharmaceutical compositions comprising arginine buffer, the use of boric acid buffer resulted in a significant higher glass corrosion and/or delamination.

59) In another embodiment, the invention relates to an aqueous pharmaceutical composition according to any one of embodiments 1) to 58), wherein the aqueous pharmaceutical composition comprises less than 10000 (preferably less than 3000, more preferably less than 500) particles with a particle size ≥ 10µm per millilitre of the aqueous pharmaceutical composition after storage of the aqueous pharmaceutical composition at 70°C for 6 days in a glass syringe.

The amount and size of the particles may be measured for instance using micro flow imaging (MFI) (alternatively called flow imaging microscopy (FIM) or dynamic imaging analysis (DIA)) as described in the experimental part.

60) In another embodiment, the invention relates to an aqueous pharmaceutical composition according to embodiment 59), wherein the glass syringe is a siliconized glass syringe.

In siliconized glass syringes the silicone is covalently bound to the inner surface of the glass barrel, for instance by plasma treatment.
61) In another embodiment, the invention relates to a container comprising (especially containing) an aqueous pharmaceutical composition according to any one of embodiments 1) to 60).
62) In another embodiment, the invention relates to a container according to embodiment 61), wherein the container is selected from a vial, an ampoule, a cartridge, or a syringe.
63) In another embodiment, the invention relates to a container according to embodiment 61), wherein the container is a syringe.
64) In another embodiment, the invention relates to a container according to any one of embodiment 61) to 63), wherein the container is a glass container (preferably a glass syringe).
65) In another embodiment, the invention relates to a container according to any one of embodiment 61) to 64), wherein the container has an inner volume between 0.1 mL and 5.0 mL. Lower limits of the inner volume are 0.1 mL, 0.3 mL, 0.5 mL and 0.8 mL, upper limits are 5.0 mL, 3.0 mL, 2.3 mL and 1.0 mL. It is to be understood that each lower limit can be combined with each upper limit. Hence all combinations shall herewith be disclosed.
66) In another embodiment, the invention relates to a container according to any one of embodiment 61) to 64), wherein the container has an inner volume between 0.5 mL and 2.3 mL.
67) In another embodiment, the invention relates to a container according to any one of embodiment 61) to 66) for use in an autoinjector device (especially a pen-injector device).
68) A further embodiment relates to a method for the preparation of an aqueous pharmaceutical composition; said method comprising the steps of:
   a) dissolving the pharmaceutically acceptable buffer (i.e. the basic component of the pharmaceutically acceptable buffer, the acidic component of the pharmaceutically acceptable buffer or any mixture thereof) and COMPOUND, or a pharmaceutically acceptable salt thereof, in water; and
   b) adjusting the pH value of the aqueous pharmaceutical composition to a pH value between 8.2 and 12.0.
69) A further embodiment relates to a method according to embodiment 68), wherein the pharmaceutically acceptable buffer is selected from arginine buffer or boric acid buffer.
70) A further embodiment relates to a method according to any one of embodiments 68) or 69), wherein water is water for injection.
71) A further embodiment relates to a method according to any one of embodiments 68) to 70), wherein the pH value is between 8.5 and 11.0.
72) A further embodiment relates to a method according to any one of embodiments 68) to 70), wherein the pH value is between 8.7 and 9.5.
73) A further embodiment relates to a method according to any one of embodiments 68) to 70), wherein the pH value is between 8.9 and 9.3.
74) A further embodiment relates to a method according to any one of embodiments 68) to 73), wherein the pH value is adjusted with a pharmaceutically acceptable strong base (and especially with an aqueous solution of sodium hydroxide).
75) A further embodiment relates to a method for the preparation of container according to any of embodiments 61) to 67), wherein the aqueous pharmaceutical composition is filled in the container using aseptic processing.

The term "aseptic processing" refers to the sterile filtration and subsequent filling of a pharmaceutical composition (especially an aqueous pharmaceutical composition) into a sterilized container under sterile conditions. The advantage of an aseptic processing is the avoidance of thermal stress of the aqueous pharmaceutical composition and its ingredients due to a thermal sterilization (such as for instance a steam sterilization) of the finally filled container.

The aqueous pharmaceutical composition according to the invention may be used as a medicament.
76) In one embodiment, the aqueous pharmaceutical composition according to the invention may be used for the preparation of a medicament, and / or is suitable, for use in the prevention/prophylaxis or treatment (especially treatment) of a disease or disorder, wherein the disease or disorder is selected from acute arterial thromboses or acute venous thromboses.
77) In a further embodiment, the aqueous pharmaceutical composition according to the invention may be used for the preparation of a medicament, and / or is suitable, for use in the prevention/prophylaxis or treatment (especially treatment) of a disease or disorder, wherein the disease or disorder is selected from acute arterial thromboses.
78) In a further embodiment, the aqueous pharmaceutical composition according to the invention may be used for the preparation of a medicament, and / or is suitable, for use in the prevention/prophylaxis or treatment (especially treatment) of a disease or disorder, wherein the disease or disorder is selected from acute coronary syndromes, percutaneous intervention (PCI) complications, stent thrombosis, periprocedural thrombotic events, myocardial infarction (especially acute myocardial infarction), peripheral ischaemia, amaurosis, sudden cardiac death, ischaemic stroke or transient ischaemic attack.
79) In a further embodiment, the aqueous pharmaceutical composition according to the invention may be used for the preparation of a medicament, and / or is suitable, for use in the prevention/prophylaxis or treatment (especially treatment) of a disease or disorder, wherein the disease or disorder is selected from acute coronary syndromes, myocardial infarction (especially acute myocardial infarction), peripheral ischaemia, amaurosis, ischaemic stroke or transient ischaemic attack.
80) In a further embodiment, the aqueous pharmaceutical composition according to the invention may be used for the preparation of a medicament, and / or is suitable, for use in the prevention/prophylaxis or treatment (especially treatment) of acute coronary syndromes.
81) In a further embodiment, the aqueous pharmaceutical composition according to the invention may be used for the preparation of a medicament, and / or is suitable, for use in the prevention/prophylaxis or treatment (especially treatment) of myocardial infarction.
82) In a further embodiment, the aqueous pharmaceutical composition according to the invention may be used for the preparation of a medicament, and / or is suitable, for use in the prevention/prophylaxis or treatment (especially treatment) of acute myocardial infarction.
83) In a further embodiment, the aqueous pharmaceutical composition according to the invention may be used for the preparation of a medicament, and / or is suitable, for use in the prevention/prophylaxis or treatment (especially treatment) of peripheral ischaemia.
84) In a further embodiment, the aqueous pharmaceutical composition according to the invention may be used for the preparation of a medicament, and / or is suitable, for use in the prevention/prophylaxis or treatment (especially treatment) of amaurosis.
85) In a further embodiment, the aqueous pharmaceutical composition according to the invention may be used for the preparation of a medicament, and / or is suitable, for use in the prevention/prophylaxis of sudden cardiac death.
86) In a further embodiment, the aqueous pharmaceutical composition according to the invention may be used for the preparation of a medicament, and / or is suitable, for use in the prevention/prophylaxis or treatment (especially treatment) of a disease, wherein the disease is selected from ischaemic stroke or transient ischaemic attack.
87) In a preferred embodiment, the aqueous pharmaceutical composition according to the invention may be used for the preparation of a medicament, and / or is suitable, for use in the emergency treatment of suspected acute coronary syndromes (ACS) or suspected acute myocardial infarction (AMI) by patient self-administration prior to hospitalization.

The term "acute coronary syndromes" (ACS) refers to syndromes due to sudden decrease or interruption of blood flow in some coronary arteries. Acute coronary syndromes encompass ST elevation myocardial infarction (STEMI), non ST elevation myocardial infarction (NSTEMI) and unstable angina. It is understood that an aqueous pharmaceutical composition that is disclosed to be useful in the prevention or treatment of ACS is likewise useful in the prevention or treatment of STEMI, NSTEMI and/or unstable angina.

The term "emergency treatment of suspected acute coronary syndromes" or "emergency treatment of suspected acute myocardial infarction" refers to a treatment of a patient wherein the patient shows symptoms of ACS or AMI, respectively, such as suddenly occurring chest pain, chest discomfort (intermittent or not), persistent retrosternal pressure or heaviness radiating to the left arm, neck, back or jaw lasting for at least 10 min, nausea/vomiting, shortness of breath, fatigue, palpitations, lightheadedness or syncope (and especially clear symptoms of ACS/AMI such as suddenly occurring chest pain, chest discomfort (intermittent or not), or persistent retrosternal pressure or heaviness radiating to the left arm, neck, back or jaw lasting for at least 10 min); and wherein the patient is to be treated and/or requires treatment before an electrocardiogram, a chest X-ray and/or blood tests could be performed. In one embodiment the patient is a patient who was already known to have a high risk to suffer from ACS/AMI before the symptoms (especially clear symptoms) of ACS/AMI occurred, such as for instance a patient with a known coronary artery disease who had a prior symptomatic episode of acute coronary syndromes/acute myocardial infarction. In a further embodiment, the treatment is effected by patient self-administration before hospitalization; it is preferred that the patient has received a training by a health care professional to better assess the symptoms of ACS/AMI before any such self-administration.

The term "coronary artery disease" (CAD) refers to a group of diseases that includes myocardial infarction, unstable angina, stable angina and sudden cardiac death.
88) In a preferred embodiment, the aqueous pharmaceutical composition according to the invention may be used for the preparation of a medicament, and / or is suitable, for use in the emergency treatment of suspected acute myocardial infarction (AMI) by patient self-administration prior to hospitalization.
89) In a further embodiment, the aqueous pharmaceutical composition according to the invention may be used for the preparation of a medicament, and / or is suitable, for use in the prevention/prophylaxis or treatment of a disease, wherein the prevention and/or prophylaxis of the formation of platelet thrombi is indicated and/or required.
90) In a further embodiment, the aqueous pharmaceutical composition according to the invention may be used for the preparation of a medicament, and / or is suitable, for use in the prevention/prophylaxis or treatment of a disease, wherein the accelerated dissolution of newly formed platelet thrombi is indicated and/or required.
91) In a further embodiment, the aqueous pharmaceutical composition according to the invention may be used for the preparation of a medicament, and / or is suitable, for use in the treatment of a disease, wherein the reduction of the size of existent platelet thrombi is indicated and/or required.

For avoidance of any doubt, if an aqueous pharmaceutical composition is described as useful for the preparation of a medicament for the prevention/prophylaxis or treatment of certain diseases or disorders, or as suitable for use in the prevention or treatment of certain diseases or disorders, such pharmaceutical composition is likewise suitable for use in a method of preventing or treating said diseases or disorders comprising administering to a subject (notably a mammal, especially a human) in need thereof a pharmaceutically active amount of said aqueous pharmaceutical composition.

The aqueous pharmaceutical composition according to the invention is suitable for parenteral administration (such as subcutaneous or intradermal administration); especially for subcutaneous administration; and notably for subcutaneous administration by patient self-administration using an autoinjector device (especially a pen-injector device).
92) In a further embodiment, the aqueous pharmaceutical composition according to the invention is administered and/or is to be administered by parenteral administration.
93) In a further embodiment, the aqueous pharmaceutical composition according to the invention is administered and/or is to be administered by subcutaneous or intradermal administration.
94) In a further embodiment, the aqueous pharmaceutical composition according to the invention is administered and/or is to be administered by subcutaneous administration.
95) In a further embodiment, the aqueous pharmaceutical composition according to the invention is administered and/or is to be administered by a health care provider.
96) In a further embodiment, the aqueous pharmaceutical composition according to the invention is administered and/or is to be administered by patient self-administration.
97) In a further embodiment, the aqueous pharmaceutical composition according to the invention is administered and/or is to be administered in a prehospital treatment.
98) In a further embodiment, the aqueous pharmaceutical composition according to the invention is administered and/or is to be administered by patient self-administration using an autoinjector device (especially a pen-injector device) in a prehospital treatment.

The chemical stability of the aqueous pharmaceutical composition may be tested in conventional manner, e.g. by measurement of COMPOUND and its degradation products (such as especially (R)-2-(6-((S)-3-methoxypyrrolidin-1-yl)-2-phenylpyrimidine-4-carboxamido)-3-phosphonopropanoic acid). The amounts of COMPOUND and its degradation products in a sample may be for instance evaluated via reversed-phase HPLC.

The aqueous pharmaceutical composition according to any one of embodiments 1) to 60) may be formulated in containers. For example, a batch size corresponding to 320g of COMPOUND (corresponding to 339g COMPOUND hydrochloride; batch size of 10.15 kg of aqueous pharmaceutical composition), may lead to at least 13000 syringes (especially glass syringes) of about 0.5 g of aqueous pharmaceutical composition per syringe. Starting from COMPOUND hydrochloride, the following amounts of ingredients may be used:

| **Material** | **Function** | **Percentage (%w/w)** | **Unit Dose** |
|---|---|---|---|
| COMPOUND hydrochloride | API | 3.34% | 16.9mg |
| L-arginine | buffer | 1.03% | 5.2mg |
| NaOH | base | 0.45% | 2.3mg |
| NaCl | tonicity modifier | 1.73% | 8.8mg |
| Water for injection (WFI) | solvent | 93.46% | 474.3mg |
| *Total* | | *100.00%* | *507.5mg (corresponding to 0.50mL)* |

The amounts may be adjusted for the purity of the respective ingredient / material. The amount of COMPOUND hydrochloride (16.9mg) corresponds to 16.0mg COMPOUND. In case COMPOUND is used in free form (non-salt form) or in form of another salt (such as for instance the disodium salt), the amount of COMPOUND in the respective form may be adjusted such that the amount of COMPOUND in the unit dose is 16.0mg; in addition, in case another form of COMPOUND than COMPOUND hydrochloride is used, the above amounts of NaOH, NaCl and water for injection may be adjusted to compensate for a different amount of NaOH needed for pH adjustment.

It is understood that the amount of COMPOUND in the unit dose may vary within the limits accepted by the health authorities and especially within a range of 16.0mg ± 1.6mg; this variation may come along with a respective change in the ratio between the ingredients or with a respective change in the total amount of the unit dose or with a combination of both.

In relation to COMPOUND in free form (non-salt form), the aqueous pharmaceutical composition may contain the following concentrations of ingredients:

| **Material** | **Function** | **Concentration [mg/mL]** | **Amount in 0.50 mL composition (unit dose)** |
|---|---|---|---|
| COMPOUND | API | 32.0 (± 3.2) | 16.0 (± 1.6) mg |
| L-arginine | buffer | 10.4 (± 1.0) | 5.2 (± 0.5) mg |
| NaCl | tonicity modifier | 20.6 (± 3.0) | 10.3 (± 1.5) mg |
| NaOH | base | q.s. to pH = 9.1 (± 0.2) | q.s. to pH = 9.1 (± 0.2) |
| Water for injection (WFI) | solvent | q.s. to final volume | q.s. to 0.50 (± 0.02) mL |

The process for the preparation of an aqueous pharmaceutical composition as described before and filled in a container according to the present invention, may comprise the following steps:
- add specific amount (e.g. 7kg) of WFI into the reactor;
- add specific amount (e.g. 104.3g) of L-Arginine into the reactor under stirring;
- add specific amount (e.g. 175.3g) of NaCl into the reactor;
- continue stirring until all raw material is dissolved;
- while continuing stirring add specific amount of COMPOUND hydrochloride (e.g. 339g) into the reactor;
- adjust the pH value with sodium hydroxide solution (e.g. 1.178 kg of 1N NaOH solution);
- fill up with WFI to final weight (e.g. 10.15kg) under stirring;
- filtrate the solution through sterile filters (e.g. 0.2µm rated Fluorodyne II membrane filters);
- fill solution into syringes (e.g. 0.548g ±2% per syringe);
- stopper syringe.

### EXAMPLES

### Abbreviations (as used herein and in the description above):

- aq.: aqueous
- API: active pharmaceutical ingredient
- CCD: charge coupled device
- DAD: diode array detector
- DMSO: dimethyl sulfoxide
- H: hour(s)
- HPLC: high performance liquid chromatography
- M: molar/molarity
- N: normal/normality
- NMP: N-methyl-2-pyrrolidone
- PVDF: polyvinylidene difluoride
- q.s.: quantum satis (amount which is enough)
- RH: relative humidity
- SEM: scanning electron microscopy
- UV: ultraviolet
- WFI: water for injection

Raw materials may be purchased from commercial suppliers, or may be prepared by methods known in the art.

### Sample preparation for High Performance Liquid Chromatography and method used

20 µL of the respective aqueous pharmaceutical composition was filled into a HPLC vial. 980 µL water was added (dilution factor 50) prior HPLC analysis.

HPLC-UV method used:

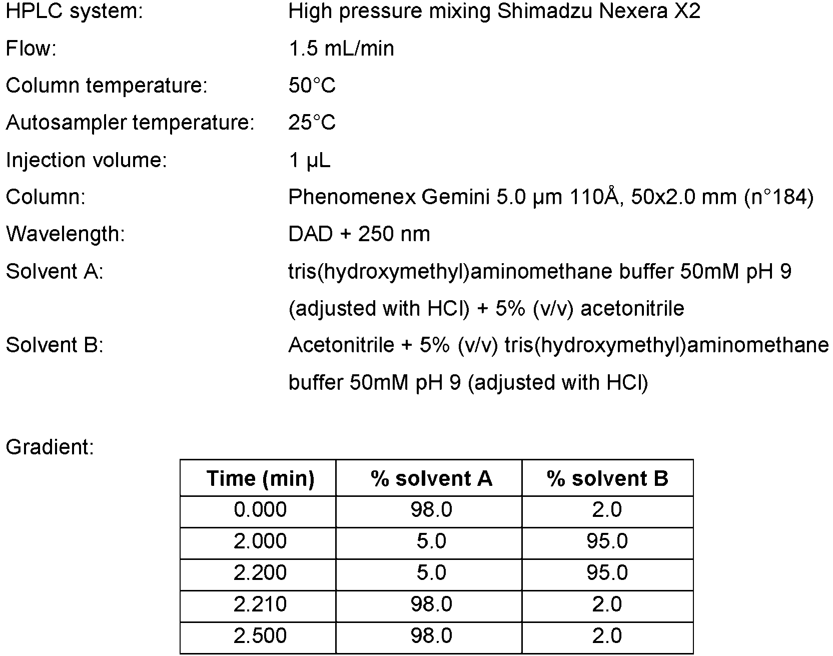

### Preparation of COMPOUND and its HCl salt:

COMPOUND and its HCl salt may be prepared according to the procedures as disclosed in WO 2009/069100 (example 2), WO 2018/055016 or Caroff E et al., J. Med. Chem. (2015), 58, 9133-9153.

### Example 1

### Preparation and composition of an aqueous pharmaceutical composition comprising an arginine buffer:

**Table 1:**

| Material | Amount |
|---|---|
| L-Arginine | 209 mg (60mM) |
| 1M Sodium Hydroxide solution | q.s. to pH 9.25 |
| COMPOUND | 640 mg (52mM) |
| Water for injection | q.s. to 20.00 mL |

### Preparation of the composition

COMPOUND and L-Arginine were weight into a 20mL volumetric flask. 10mL water for injection was added and the suspension stirred. This suspension was then titrated to pH 9.25 resulting in full dissolution. Prior to filling, the formulation was filtered through 0.22µm PVDF filter.

### Preparation of the primary packaging

0.5 mL of the sterile filtered formulation was filled into a sterile ready-to-use pre-fillable glass-syringe. The filling takes place under sterile and particle free conditions.

### Example 2

### Preparation and composition of an aqueous pharmaceutical composition comprising a boric acid buffer:

**Table 2:**

| Material | Amount |
|---|---|
| Boric acid | 3710 mg (60mM) |
| 5M Sodium Hydroxide | q.s. to pH 9.25 |
| solution | |
| COMPOUND | 32000 mg (52mM) |
| Water for injection | q.s. to 1000 mL |

### Preparation of the composition

Boric acid and COMPOUND were weight into a 1000 mL volumetric flask. 750mL water for injection was added and the suspension stirred. 30mL of NaOH solution was added and the suspension was stirred until full dissolution of the added powders. After full dissolution the pH was adjusted to pH 9.25 by adding NaOH solution. Finally, the volumetric flask was filled up with water for injection. The formulation was filtered through 0.22µm PVDF filters prior to filling 0.5mL aliquots into sterile pre-fillable glass-syringes.

### Example 3

### Preparation and composition of aqueous pharmaceutical compositions comprising additional buffers:

Additional buffers are prepared at a fixed buffer capacity of 34 mM/pH at pH 9.25.

**Table 3: Tris(hydroxymethyl)aminomethane buffer**

| Material | Amount |
|---|---|
| tris(hydroxymethyl)aminomethane | 472.4 mg (195mM) |
| 1M Sodium Hydroxide solution | q.s. to pH 9.25 |
| COMPOUND | 640 mg (52mM) |
| Water for injection | q.s. to 20 mL |

**Table 4: L-Histidine buffer**

| Material | Amount |
|---|---|
| L-Histidine | 186.2 mg (60mM) |
| 1M Sodium Hydroxide solution | q.s. to pH 9.25 |
| COMPOUND | 640 mg (52mM) |
| Water for injection | q.s. to 20 mL |

**Table 5: Glycine buffer**

| Material | Amount |
|---|---|
| Glycine | 108.1 mg (72mM) |
| 1M Sodium Hydroxide solution | q.s. to pH 9.25 |
| COMPOUND | 640 mg (52mM) |
| Water for injection | q.s. to 20 mL |

**Table 6: Meglumine buffer**

| Material | Amount |
|---|---|
| Meglumine | 281.1 mg (72mM) |
| 1M Sodium Hydroxide solution | q.s. to pH 9.25 |
| COMPOUND | 640 mg (52mM) |
| Water for injection | q.s. to 20 mL |

**Table 7: Carbonate buffer**

| Material | Amount |
|---|---|
| Sodium hydrogencarbonate (NaHCO₃) | 168.0 mg (100mM) |
| 1M Sodium Hydroxide solution | q.s. to pH 9.25 |
| COMPOUND | 640 mg (52mM) |
| Water for injection | q.s. to 20 mL |

**Table 8: Ammonium chloride buffer**

| Material | Amount |
|---|---|
| Ammonium chloride (NH₄Cl) | 64.2 mg (60mM) |
| 1M Sodium Hydroxide solution | q.s. to pH 9.25 |
| COMPOUND | 640 mg (52mM) |
| Water for injection | q.s. to 20 mL |

### Preparation of the composition

COMPOUND and the respective buffer (according to tables 3 to 8) were weight into 20mL volumetric flasks. 10 mL water for injection was added to each flask and the suspensions were stirred. These suspensions were then titrated to pH 9.25 which led to full dissolution. Prior to filling the formulations into 1mL sterile, pre-fillable glass-syringes they were filtered through 0.22µm PVDF filter.

### Example 4

### Stability of COMPOUND in aqueous solution at different pH values in the absence of a buffer

Aqueous formulations containing 32 mg/mL (52mM) COMPOUND were titrated to pH 7.0, 8.0, and 9.0 using aqueous 1N NaOH solution.

The different formulations were filled into 1mL sterile, pre-fillable glass syringes after sterile filtration. Samples were analyzed at t=0 and after 1-week storage at 60°C.

**Table 9: Content of COMPOUND and DEGRADATION COMPOUND ((R)-2-(6-((S)-3-methoxypyrrolidin-1-yl)-2-phenylpyrimidine-4-carboxamido)-3-phosphonopropanoic acid) after 1 week storage at 60°C in aqueous formulations at different pH values**

| pH value | COMPOUND [Area%] | | DEGRADATION COMPOUND [Area%] | |
|---|---|---|---|---|
| | t = 0 | t = 1 week at 60°C | t = 0 | t = 1 week at 60°C |
| 7.0 | 98.55 | 33.5 | 0.95 | 65.2 |
| 8.0 | 98.65 | 74.7 | 0.84 | 24.4 |
| 9.0 | 98.60 | 88.7 | 0.82 | 10.0 |

### Example 5

### Stability of COMPOUND in aqueous solution at different pH values in the presence or absence of a buffer

Aqueous formulations containing 32 mg/mL (52mM) COMPOUND and 20mM KH2PO4 were titrated to pH 7.0, 7.5, 8.0 using aqueous 1N NaOH solution. Aqueous formulations containing 52mM COMPOUND and 20mM boric acid were titrated to pH 8.0, 8.5, 9.0, 9.5, 10.0 using aqueous 1N NaOH solution. A formulation containing 52mM COMPOUND without added buffer was titrated to pH 9.0 using aqueous 1N NaOH solution.

The different formulations were filled into 1mL sterile, pre-fillable glass-syringes after sterile filtration.

Syringes were stored under controlled temperature at 40, 60, or 70°C for 2 weeks. The content of COMPOUND and of the main degradation product (R)-2-(6-((S)-3-methoxypyrrolidin-1-yl)-2-phenylpyrimidine-4-carboxamido)-3-phosphonopropanoic acid was determined by HPLC analysis using conditions as described above.

**Table 10: Content of COMPOUND and DEGRADATION COMPOUND ((R)-2-(6-((S)-3-methoxypyrrolidin-1-yl)-2-phenylpyrimidine-4-carboxamido)-3-phosphonopropanoic acid) after 2 weeks temperature stress in aqueous formulations at different pH values**

| | **COMPOUND [Area%]** | | | **DEGRADATION COMPOUND [Area%]** | | |
|---|---|---|---|---|---|---|
| | 40°C | 60°C | 70°C | 40°C | 60°C | 70°C |
| Without buffer (pH 9.0) | 97.73 | 84.49 | 50.02 | 2.03 | 14.60 | 47.63 |
| 20mM KH2PO4, pH 7.0 | 74.96 | 20.68 | 2.49 | 24.72 | 78.21 | 94.93 |
| 20mM KH2PO4, pH 7.5 | 85.01 | 36.88 | 11.74 | 14.85 | 62.47 | 85.15 |
| 20mM KH2PO4, pH 8.0 | 95.11 | 68.31 | 20.99 | 4.63 | 30.95 | 76.95 |
| 20mM boric acid, pH 8.0 | 95.40 | 66.16 | 27.73 | 4.43 | 33.25 | 70.45 |
| 20mM boric acid, pH 8.5 | 97.35 | 80.27 | 46.96 | 2.39 | 18.93 | 51.06 |
| 20mM boric acid, pH 9.0 | 97.83 | 85.68 | 57.79 | 1.97 | 13.03 | 39.59 |
| 20mM boric acid, pH 9.5 | 97.56 | 85.97 | 63.39 | 2.10 | 11.95 | 32.64 |
| 20mM boric acid, pH 10.0 | 97.18 | 83.13 | 62.08 | 2.39 | 13.39 | 31.80 |

### Example 6

### Stability of COMPOUND in aqueous solution at pH 9.0 in the presence of boric acid buffer at different buffer concentrations

Formulation compositions assessing the impact of buffer capacity on formulation stability

| **B(OH)₃** | | **NaOH 1M** | **COMPOUND** | | **Water** |
|---|---|---|---|---|---|
| **[mM]** | **[mg]** | | **[mM]** | **[mg/mL]** | |
| 0 | 0 | q.s. pH 9.0 | 52 | 32 | q.s. 5mL |
| 20 | 6.183 | q.s. pH 9.0 | 52 | 32 | q.s. 5mL |
| 60 | 18.549 | q.s. pH 9.0 | 52 | 32 | q.s. 5mL |
| 100 | 30.915 | q.s. pH 9.0 | 52 | 32 | q.s. 5mL |

**Table 11: Content of COMPOUND after storage of aqueous formulations containing different boric acid buffer concentrations at 75°C in dependence of the storage time**

| COMPOUND [Area%] | stored at 75°C for time [h] | | | | |
|---|---|---|---|---|---|
| c(B(OH)₃) [mM] | 0 | 24 | 48 | 120 | 168 |
| 0 | 100.00 | 96.16 | 89.46 | 64.89 | 49.45 |
| 20 | 100.00 | 94.61 | 90.72 | 74.84 | 62.18 |
| 60 | 100.00 | 96.60 | 90.21 | 78.47 | 68.96 |
| 100 | 100.00 | 97.89 | 93.78 | 77.85 | 71.33 |

### Example 7

### Stability of COMPOUND in buffered aqueous solutions at basic pH values

The syringes filled with aqueous pharmaceutical compositions as described under example 1 (L-arginine buffer), example 2 (boric acid buffer) and example 3 were stored at 55.3°C for 19 days. The content of COMPOUND and of the main degradation product (R)-2-(6-((S)-3-methoxypyrrolidin-1-yl)-2-phenylpyrimidine-4-carboxamido)-3-phosphonopropanoic acid was determined by HPLC analysis using conditions as described above.

**Table 12: Content of COMPOUND and DEGRADATION COMPOUND ((R)-2-(6-((S)-3-methoxypyrrolidin-1-yl)-2-phenylpyrimidine-4-carboxamido)-3-phosphonopropanoic acid) after 19 days storage of aqueous formulations containing different buffers at 55.3°C**

| | **COMPOUND** [Area%] | **DEGRADATION COMPOUND** [Area%] |
|---|---|---|
| 60mM L-Arginine | 88.55 | 10.02 |
| 60mM Boric acid | 86.76 | 11.01 |
| 72mM Glycine | 86.26 | 12.01 |
| 195mM Tris(hydroxymethyl)aminomethane | 86.56 | 11.26 |
| 72mM Meglumine | 87.06 | 11.16 |
| 60mM Histidine | 85.65 | 11.79 |
| 60mM Ammonium chloride | 85.05 | 13.22 |
| 100mM Carbonate | 83.56 | 13.52 |

### Example 8

### Interaction of the aqueous pharmaceutical composition with glass surfaces

The aqueous formulation of Example 2 (32mg/mL COMPOUND in 60mM boric acid buffer, pH 9.25, filled in 1mL pre-fillable glass-syringes) and of Example 1 (32mg/mL COMPOUND in 60mM Arginine buffer, pH 9.25, filled in 1mL pre-fillable glass-syringes) were each stressed at 70°C for 6 days.

The particle counts were analyzed by Micro-Flow Imaging for the two different aqueous formulations. Micro-Flow Imaging uses a CCD camera to capture bright field images in a flow cell. It has an optical magnification and detects particles in a size range from 2.0 µm to approximately 70 µm. The particles are counted and imaged.

Prior to analysis the cleanliness of the instrument was tested and a count and size standard was measured to verify that the instrument was suitable for analysis. The samples were left at ambient temperature and gently homogenized (avoiding the creation of air bubbles) before analysis. No dilution or other manipulation of the samples was performed. For the actual measurement the optimization of illumination was performed with the sample to be analyzed.

**Table 13: Particle count and particle size distribution in aqueous formulations stored in glass-syringes for 6 days at 70°C**

| cumulative particle counts | | | | | |
|---|---|---|---|---|---|
| Formulation: | ≥2µm | ≥5µm | ≥10µm | ≥25µm | ≥50µm |
| 60mM boric acid | 182254 | 61815 | 25419 | 6556 | 1364 |
| 60mM Arginine | 6466 | 405 | 18 | 4 | 0 |

The particle count, especially of larger particles, is higher in the formulation containing a boric acid buffer than in a formulation containing an arginine buffer if stored in glass syringes at 70°C for 6 days.

After storage for 6 days at 70°C (as described above), the glass barrel of the 1mL pre-fillable glass-syringes containing the boric acid formulation (Example 2) was further analyzed by Scanning Electron Microscopy (SEM):
To prevent charge loading during SEM acquisition, the sample was gold-coated using a Cressington 108 Auto sputter, set to 30 mA for 80 s under 0.1 mbar Argon flow. SEM was performed with a Zeiss EVO MA10, using a SE1 detector, with an acceleration voltage of 10 kV and an extraction current of 100 pA. Images were acquired at different magnifications. Scanning parameter were set to an averaging of 17 lines resulting in an overall frame acquisition of ca. 44s. Fig. 1 shows the SEM images of an area of the inner part of the glass barrel that was in contact with the boric acid formulation. Fig. 2 shows the SEM images of an area of the inner part of the glass barrel that was not in contact with the boric acid formulation.

The figures show that boric acid leads to glass delamination / glass corrosion in glass syringes leading to increased particle loads in the boric acid containing formulation.

### Example 9

### Long-term stability of COMPOUND in aqueous solution at pH 9.1 in the presence of 60 mM arginine buffer under different storage conditions

In a 10 L glass reactor, L-arginine (104 g, 0.60 mol) was added under stirring to water for injection (7.01 kg). COMPOUND hydrochloride (339 g, 0.52 mol) was added portion wise to the solution and the pH value was adjusted to pH = 9.0 by addition of 1.0 M aqueous NaOH solution (1.18 kg). Water for injection was added to a total weight of the solution of 10.15 kg.

For stability testing the test samples were prepared by filling 0.54 mL of the above solution into prefillable 1 mL glass syringes with stacked in needle and stored under the conditions given in table 14 and 15. The samples were analysed after the given timepoints by ultra-performance liquid chromatography (UPLC) using the following conditions:

### Equipment:

UPLC system, with data acquisition system and UV detection
Mobile phase A: Weigh 3.08 g of ammonium acetate and 3.26 g of ammonium hexafluorophosphate in a 2L bottle. Dissolve in 1900 mL H2O. Add 100mL of acetonitrile.
Mobile phase B: Weigh 0.77 g of ammonium acetate in a 1L bottle. Dissolve in 50 mL H2O. Add 950mL of acetonitrile.
Diluent: Mix 920mL of Mobile Phase A with 80mL Mobile phase B.
Reference standard solution: 0.32 mg/mL of COMPOUND reference standard in diluent.
Sample solution: The samples were diluted to 0.32 mg/mL of COMPOUND (based on amount of COMPOUND in the initial solution) with diluent
Column: 100mm x 3.0 mm, 3 µm C18 column (e.g. YMC Triart C18 ExRS)
Detection wavelength: 246 nm
Flow rate: 0.5 mL/min
Gradient:

| Time [min] | % A | % B |
|---|---|---|
| Initial | 92 | 8 |
| 14 | 50 | 50 |
| 18 | 0 | 100 |
| 19 | 0 | 100 |
| 19.1 | 92 | 8 |
| 23 | 92 | 8 |

**Table 14: Amount of COMOUND in the test samples after storage for up to 12 months under the given conditions**

| COMPOUND [mg] | initial | 1 month | 3 months | 6 months | 12 months |
|---|---|---|---|---|---|
| 5°C | 16.9 | 16.9 | 16.8 | 16.6 | |
| 25°C / 60%RH | 16.9 | 16.8 | 16.4 | 16.2 | 15.9 |
| 30°C / 75%RH | 16.9 | 16.7 | 16.2 | 15.9 | 15.2 |
| 40°C / 75%RH | 16.9 | 16.2 | 15.1 | 13.3 | |

**Table 15: Amount of DEGRADATION COMPOUND ((R)-2-(6-((S)-3-methoxypyrrolidin-1-yl)-2-phenylpyrimidine-4-carboxamido)-3-phosphonopropanoic acid) in the test samples after storage for up to 12 months under the given conditions**

| DEGRADATION COMPOUND [Area%] | initial | 1 month | 3 months | 6 months | 12 months |
|---|---|---|---|---|---|
| 5°C | 0.38 | 0.41 | 0.57 | 0.58 | |
| 25°C / 60%RH | 0.41 | 0.59 | 1.19 | 1.72 | 3.06 |
| 30°C /75%RH | 0.41 | 0.77 | 1.86 | 3.0 | 5.8 |
| 40°C / 75%RH | 0.41 | 2.2 | 7.0 | 12.4 | |

## Claims

1. An aqueous pharmaceutical composition comprising:
• 4-((R)-2-{[6-((S)-3-methoxy-pyrrolidin-1-yl)-2-phenyl-pyrimidine-4-carbonyl]-amino}-3-phosphono-propionyl)-piperazine-1-carboxylic acid butyl ester;
• a pharmaceutically acceptable buffer; and
• water;
wherein the aqueous pharmaceutical composition has a pH value between 8.2 and 12.0.

2. An aqueous pharmaceutical composition according to claim 1, wherein the sum of the amount of 4-((R)-2-{[6-((S)-3-methoxy-pyrrolidin-1-yl)-2-phenyl-pyrimidine-4-carbonyl]-amino}-3-phosphono-propionyl)-piperazine-1-carboxylic acid butyl ester, of the amount of the pharmaceutically acceptable buffer and of the amount of water is at least 95 ww% of the aqueous pharmaceutical composition.

3. An aqueous pharmaceutical composition according to claim 1, consisting essentially of:
• 4-((*R*)-2-{[6-((*S*)-3-methoxy-pyrrolidin-1-yl)-2-phenyl-pyrimidine-4-carbonyl]-amino}-3-phosphono-propionyl)-piperazine-1-carboxylic acid butyl ester in an amount between 0.7 to 7.0 ww%;
• a pharmaceutically acceptable buffer selected from a boric acid buffer or an arginine buffer;
• water; and
• optionally an inorganic salt of an alkali metal or an alkaline earth metal;
wherein the aqueous pharmaceutical composition has a pH value between 8.7 and 9.5.

4. An aqueous pharmaceutical composition according to any one of claims 1 or 2, wherein the mass concentration of 4-((*R*)-2-{[6-((*S*)-3-methoxy-pyrrolidin-1-yl)-2-phenyl-pyrimidine-4-carbonyl]-amino}-3-phosphono-propionyl)-piperazine-1-carboxylic acid butyl ester in the aqueous pharmaceutical composition is between 6 mg/mL and 60 mg/mL.

5. An aqueous pharmaceutical composition according to any one of claims 1, 2 or 4, wherein the pharmaceutically acceptable buffer is selected from ammonium buffer, boric acid buffer, carbonate buffer, phosphate buffer, arginine buffer, glycine buffer, histidine buffer, meglumine buffer, or tris(hydroxymethyl)aminomethane buffer.

6. An aqueous pharmaceutical composition according to any one of claims 1 to 5, wherein the aqueous pharmaceutical composition has a pH value between 8.7 and 9.5.

7. An aqueous pharmaceutical composition according to any one of claims 1 to 6, wherein the aqueous pharmaceutical composition comprises sodium chloride.

8. An aqueous pharmaceutical composition according to any one of claims 1 to 7, wherein the osmolality of the aqueous pharmaceutical composition is between 230 mOsm/kg and 1000 mOsm/kg.

9. An aqueous pharmaceutical composition according to any one of claims 1 to 8, wherein the aqueous pharmaceutical composition is in a container and wherein the container is selected from a vial, an ampoule, a cartridge, or a syringe.

10. An aqueous pharmaceutical composition according to any one of claims 1 to 9, wherein the amount of 4-((R)-2-{[6-((S)-3-methoxy-pyrrolidin-1-yl)-2-phenyl-pyrimidine-4-carbonyl]-amino}-3-phosphono-propionyl)-piperazine-1-carboxylic acid butyl ester in the aqueous pharmaceutical composition after storage for 1 year at 25°C is at least 80% of the amount of 4-((R)-2-{[6-((S)-3-methoxy-pyrrolidin-1-yl)-2-phenyl-pyrimidine-4-carbonyl]-amino}-3-phosphono-propionyl)-piperazine-1-carboxylic acid butyl ester in the aqueous pharmaceutical composition immediately after final preparation of the aqueous pharmaceutical composition.

11. A container comprising an aqueous pharmaceutical composition according to any one of claims 1 to 10, wherein the container is selected from a vial, an ampoule, a cartridge, or a syringe.

12. A method for the preparation of a container according to claim 11, wherein the aqueous pharmaceutical composition is filled in the container using aseptic processing.

13. An aqueous pharmaceutical composition according to any one of claims 1 to 10 for use in the prevention or treatment of a disease or disorder, wherein the disease or disorder is selected from acute arterial thromboses or acute venous thromboses.

14. An aqueous pharmaceutical composition according to any one of claims 1 to 10 for use in the prevention or treatment of acute myocardial infarction.

15. An aqueous pharmaceutical composition according to any one of claims 1 to 10 for use in the emergency treatment of suspected acute myocardial infarction by patient self-administration prior to hospitalization.

16. An aqueous pharmaceutical composition according to any one of claims 1 to 10 for use according to any one of claims 13 to 15, wherein the aqueous pharmaceutical composition is administered and/or is to be administered by subcutaneous administration.
